# EUROPEAN PATENT SPECIFICATION

(11) **EP 2 402 020 B1**
(45) Date of publication and mention of the grant of the patent: **04.12.2013**
(21) Application number: 10168143.5
(22) Date of filing: 01.07.2010
(51) Int. Cl.: A61K 36/233, A61P 29/00, A61P 37/00, A61P 37/06, A61P 37/08, A61P 17/00, A61P 27/00, A61P 9/00, A61P 11/00, A61P 11/06, A61P 1/00

(54) **Pharmaceutical composition comprising extracts of Bupleurum krylovianum for use in the treatment of arthritis and rheumatoid arthritis**
Pharmazeutische Zusammensetzung enthaltend Bupleurum krylovianum Extrakte zur Behandlung der Arthritis und der rheumatoide Arthritis
Composition pharmaceutique comprenant des extraits de Bupleurum krylovianum pour le traitement de l'arthrite et de la polyarthrite rhumatoïde

(43) Date of publication of application: 04.01.2012
(73) Proprietor: Industrial Technology Research Institute, Hsinchu 31040 (TW)
(72) Inventor: Shih, Ying-Chu, Hsinchu County, Taiwan 302 (CN); Lee, Lain-Tze, Hsinchu City, Taiwan 300 (CN); Tsai, Bie-Shung, Taipei, Taiwan 100 (CN); Lo, Jir-Mehng, Hsinchu County, Taiwan 306 (CN); Tong, Tien-Soung, Hsinchu, Taiwan 300 (CN); Sheu, Jenn-Line, Hsinchu, Taiwan 300 (CN); Huang, Kuo-Kuei, Hsinchu County, Taiwan 310 (CN); Tsai, Ying-Fei, Hsinchu, Taiwan 300 (CN); Lee, Yi-Ching, Hsinchu, Taiwan 300 (CN); Liang, Hui-Ju, Taipei County, Taiwan 100 (CN); Yen, Jui-Hung, Taipei County, Taiwan 247 (CN); Lee, Cheng-Yu, Hsinchu, Taiwan 300 (CN)
(74) Representative: Fuchs

(56) References cited:
- WON SICK WOO ET AL: "Antiinflammatory principle of Bupleurum longiradiatum roots", ARCHIVES OF PHARMACAL RESEARCH, vol. 16, no. 1, 1 January 1993 (1993-01-01), pages 29-31, XP009140751, NATL. FISHERIES UNIVERSITY, PUSAN, KR ISSN: 0253-6269
- DATABASE WPI Week 200805 Thomson Scientific, London, GB; AN 2008-A60306 XP002609610, & CN 1 994 378 A (CHANGSHU LEIYUNSHANG PHARMACY CO LTD) 11 July 2007 (2007-07-11)
- CHENG X Q ET AL: "Macrophage immunomodulatory activity of the polysaccharides from the roots of Bupleurum smithii var. parvifolium", JOURNAL OF ETHNOPHARMACOLOGY, vol. 130, no. 2, 20 July 2010 (2010-07-20) , pages 363-368, XP027148200, ELSEVIER SCIENTIFIC PUBLISHERS LTD, IE ISSN: 0378-8741 [retrieved on 2010-07-16]
- GONZALEZ J A ET AL: "BIOLOGICAL ACTIVITY OF SECONDARY METABOLITES FROM BUPLEURUM SALICIFOLIUM (UMBELLIFERAE)", EXPERIENTIA, vol. 51, no. 1, 1 January 1995 (1995-01-01), pages 35-39, XP008051894, BIRKHAEUSER VERLAG. BASEL, CH ISSN: 0014-4754
- GONZALEZ ET AL: "Isokaerophyllin, a butyrolactone from Bupleurum salicifolium", PHYTOCHEMISTRY, vol. 29, no. 2, 1 January 1990 (1990-01-01), pages 675-678, XP022515638, PERGAMON PRESS, GB ISSN: 0031-9422
- YANG Z-Y ET AL: "ITS sequence analysis used for molecular identification of the Bupleurum species from northwestern China", PHYTOMEDICINE, vol. 14, no. 6, 27 June 2007 (2007-06-27), pages 416-423, XP025320950, GUSTAV FISCHER VERLAG, STUTTGART, DE ISSN: 0944-7113, DOI: 10.1016/J.PHYMED.2007.04.009 [retrieved on 2007-05-30]
- CHANG W-L ET AL: "Immunosuppressive flavones and lignans from Bupleurum scorzonerifolium", PHYTOCHEMISTRY, vol. 64, no. 8, 1 December 2003 (2003-12-01), pages 1375-1379, XP004473110, PERGAMON PRESS, GB ISSN: 0031-9422, DOI: 10.1016/J.PHYTOCHEM.2003.08.002
- Anonymous: "Altai Bupleurum", , [Online] 9 November 2010 (2010-11-09), XP002609611, Wikipedia, der freien Enzyklopädie Retrieved from the Internet: URL:http://zh.wikipedia.org/zh-hant/%E9%98 %BF%E5%B0%94%E6%B3%B0%E6%9F%B4%E8%83%A1> [retrieved on 2010-11-15] -& Anonymous: "Altai Bupleurum", , 9 November 2010 (2010-11-09), XP002609612, Wikipedia, der freien Enzyklopädie Retrieved from the Internet: URL:http://translate.google.de/translate?h l=de&sl=auto&tl=de&u=http%3A%2F%2Fzh.wikip edia.org%2Fzh-hant%2F%25E9%2598%25BF%25E5% 25B0%2594%25E6%25B3%25B0%25E6%259F%25B4%25 E8%2583%25A1> [retrieved on 2010-11-15]
- YANG YAO-JUN ET AL: "Flavonoids in Altai Chaihu (Altai Bupleurum Root)", BEIJING ZHONGYIYAO DAXUE XUEBAO - JOURNAL OF BEIJING UNIVERSITYOF TRADITIONAL CHINESE MEDICINE, vol. 33, no. 3, 1 March 2010 (2010-03-01), pages 207-209, XP009140958, GAI-KAN BIANJIBU, BEIJING, CN ISSN: 1006-2157
- YAOJUN ET AL: "Chemical components in volatile oil from Altai Bupleurum Root", BEIJING ZHONGYIYAO DAXUE XUEBAO - JOURNAL OF BEIJING UNIVERSITYOF TRADITIONAL CHINESE MEDICINE, vol. 28, 1 November 2005 (2005-11-01), pages 63-65, XP009140955, GAI-KAN BIANJIBU, BEIJING, CN ISSN: 1006-2157
- Menglan, Watson: "Bupleurum", , [Online] vol. 14, 2005, pages 60-74, XP002609613, Flora of China Retrieved from the Internet: URL:http://flora.huh.harvard.edu/china/PDF /PDF14/Bupleurum.pdf> [retrieved on 2010-11-15]

<110> Industrial Technology Research Institute
<120> PHARMACEUTICAL COMPOSITION WITH IMMUNOMODULATING FUNCTION
<130> TT 0090 EP
<160> 6
<210> 1
   <211> 629
   <212> DNA
   <213> Bupleurum
<400> 1
   ggatcattgt cgaatcctga aacgaagagc gacccgagaa catgttttaa gacggggcca 60
   gcggtsgtcg gcctcggcct gacggctgcg aaccctaggc cggggggctc ctagttgtgc 120
   ccgccggccc aaaacctaac cgggcgcgga atgcgccaag gaaaccgaaa ctgaacagga 180
   tgtctccgcc ccgtttgagg gggggtcgac atccttctga gaaacaaacg actctcggca 240
   acggatatcc cggctctcgc atcgatgaag aacgtagcga aatgcgatac ttggtgtgaa 300
   ttgcagaatc ccgtgaacca tcgagttttt gaacgcaagt tgcgcccgat gccattaggc 360
   tgagggcacg tctgcctggg tgtcacgtaa agctttgccc ctccgcagct cgctcaaagc 420
   gagtcggtgc tgttcggggg gacggaaatt gacctcccgt gcctcgtcgt gcggctggtt 480
   taaaagagag tctccggaga tcggaaaacg caacattggt ggaatgcatt acgcacctct 540
   tgccatcttg cgctgagccc gtttactctg tgagcaacag cgaccctttg gcgccgcccc 600
   aggtgcgcgc tcgaactgtg accccaggt                                   629
<210> 2
   <211> 629
   <212> DNA
   <213> Bupleurum
<400> 2
   ggatcattgt cgaatcctga atcgaagtgc gacccgagaa catgttttaa gacggggcca 60
   gcggtcgtcg gcctcggcct gacggctgcg aaccctaggt cggggggcgc ctagttgtgc 120
   ctgccggccc aaaacctaac cgggcgcgga atgcgccaag gaaaccgaaa ctgaacagga 180
   tgtctccgcc ccgtttgagg gggggtcatc atccttctga gaaacaaacg actctcggca 240
   acggatatcc cggctctcgc atcgatgaag aacgtagcga aatgcgatac ttggtgtgaa 300
   ttgcagaatc ccgtgaacca tcgagttttt gaacgcaagt tgcgcccgat gccattaggc 360
   tgagggcacg tctgcctggg tgtcacgtaa agctttgccc ctccgcagct cgctcaaagc 420
   gagtcgttga tgttcggggg gacggaaagt gacctcccgt gcctcgtcgt gcggctggtt 480
   taaaagagag tctccggaga tcggaaaacg caacattggt ggaaggcatt acgcacctct 540
   tgccatcttg cgctgagccc gtttactcca tgagcaacag cgaccctttg gcgccgcccc 600
   aggtgagcgc tcgaactgtg accccaggt                                   629
<210> 3
   <211> 629
   <212> DNA
   <213> Bupleurum
<220>
   <221>
   <222> 107
   <223> unspecified nucleotide, n is any of g, a, t, or c
<400> 3
   ggatcattgt cgaatcctga atcgaagagc gacccgagaa catgttttaa gacggggcca 60
   gcggtcgtcg gcctcggcct gacggctgcg aaccctaggc cgggggntgc ctagttgtgc 120
   ccgccggccc aaaacctaac cgggcgcgga atgcgccaag gaaaccgaaa ctgaacagga 180
   tgtctccgcc ccgtttaagg gggggtcgac atccttctga gaaacaaacg actctcggca 240
   acggatatcc cggctctcgc atcgatgaag aacgtagcga aatgcgatac ttggtgtgaa 300
   ttgcagaatc ccgtgaacca tcgagttttt gaacgcaagt tgcgcccgat gycattaggc 360
   tgagggcacg tctgcctggg tgtcacgtaa agctttgccc ctccgcagct cgctcaaatc 420
   gagtcgttgc tgttcggggg gacggaaatt gacctcccgt gcctcgtcgt gcggctggtt 480
   taaaagagag cctccggaga tcggaaaacg caacattggt ggaaggcatt acgcacctct 540
   tgccatcttg cgctgagccc gttyactctg tgagcaacag cgaccctttg gcgccgcccc 600
   aggcgcgcgc tcgaactgtg accccaggt                                   629
<210> 4
   <211> 629
   <212> DNA
   <213> Bupleurum
<400> 4
   ggatcattgt cgaatcctga atcgaagacc gacccgagaa catgttttaa gacggggcca 60
   gcggtcgtcg gcctcggcct gacggctgcg aaccctaggc cggggggcgc ctagttgtgc 120
   ccgccggccc aaaacctaac cgggcgcgga atgcgccaag gaaaccgaaa ctgaacagga 180
   tgtctccgcc ccgtttgagg gggggtcgac atccttcaaa gaaacaaacg actctcggca 240
   acggatatcc cggctctcgc atcgatgaag aacgtagcga aatgcgatac ttggtgtgaa 300
   ttgcagaatc ccgtgaacca tcgagttttt gaacgcaagt tgcgcccgat gccattaggc 360
   tgagggcacg tctgcctggg tgtcacgtat agctttgccc ctccgcagct cgctcaaagc 420
   gagtcgttgc tgttcggggg gacggaaatt gacctcccgt gcctcgtcgt gcggctggtt 480
   taaaagagag tctccggaga tcggaaaacg caacattggt ggaatgcatt acgcacctct 540
   tgccatcttg cgctgagccc gtttactctg tgagcaacag cgaccctttg gcgccgcccc 600
   aggtgcgcgc tcgaactgtg accccaggt                                   629
<210> 5
   <211> 629
   <212> DNA
   <213> Bupleurum
<400> 5
   ggatcattgt cgaatcctga atcgaagagc gacccgagaa catgttttaa gacggggcca 60
   gcggtcgtcg gcctcggcct gacgtctgcg aaccctaggc cggggggcgc ctagttgtgc 120
   ccgccggccc aaaacctaac cgggcgcgga atgcgccaag gaaaccgaaa ctgaacagga 180
   tgtctccgcc ccgtttgagg gggggtcgac atccttctga gaaacaaacg actctcggca 240
   acggatatcc cggctctcgc atcgatgaag aacgtagcga aatgcgatac ttggtgtgaa 300
   ttgcagaatc ccgtgaacca tcgagttttt gaacgcaagt tgcgcccgat gccattaggc 360
   tgagggcacg tctgcctggg tgtcacgtaa agctttgccc ctccgcagct cgctcgaagc 420
   gagtcgttgc tgttcggggg gacggaaagt gacctcccgt gcctcgtcgt gcggctggtt 480
   taaaagagag cctccggaga tcggaaaacg caacattggt ggaaggcatt acgcacctct 540
   tgccatcttg cgctgagccc gtttactctg tgagcaactg cgaccctttg gcgccgcccc 600
   aggtgcgcgc tcgaactgtg accccaggt                                   629
<210> 6
   <211> 629
   <212> DNA
   <213> Bupleurum
<400> 6
   ggatcattgt cgaatcctga atcgaagagc gacccgagaa catgttttaa gacggggcca 60
   gcggtcgtcg gcctcggcct gacggctgcg aaccctaggc cggggggcgc ctagttgtgc 120
   ccgccgaccc aaaacctaac cgggcgcgga atgcgccaag gaaaccgaaa ctgaacagga 180
   tgtctccgcc cctttcgagg gggggtcgac atccttctga aaaacaaacg actctcggca 240
   acggatatcc cggctctcgc atcgatgaag aacgtagcga aatgcgatac ttggtgtgaa 300
   ttgcagaatc ccgtgaacca tcgagttttt gaacgcaagt tgcgcccgat gccattaggc 360
   cgagggcacg tctgcctggg tgtcacgtaa agctttgccc ctccgcagct cgctcaaagc 420
   gagtcgttgc tctcgtgggg gacggaaatt gacctcccgt gcctcgtcgt gcggctggtt 480
   taaaagagag tctccgggga tcggaaaacg caacattggt ggaaggcatt acgcacctct 540
   tgccatcttg cgctgagccc gtttactctg tgagcaacag cgaccctttg gcgccgcccc 600
   aggtgcgcgc tcgaactgtg accccaggt                                  629

## Description

### BACKGROUND

### Technical Field

The present specification relates to a pharmaceutical composition with an immunomodulating function, and in particular relates to an extract of Bupleurum with an immunomodulating function.

### Description of the Related Art

The mechanism for immune disorders has been reported in several studies. Many studies indicate that the over-expressed tumor necrosis factor (TNF-α) is related to immuno-inflammatory diseases, such as rheumatoid arthritis (RA), Crohn's disease, psoriatic arthritis or ankylosing spondylitis.

TNF-α functions like cytokines, modulates cell recruitment, cell proliferation, cell death and immune regulation. At low concentrations in tissues, TNF-α may augment host defense mechanisms against infection. At high concentrations, TNF-α results in excess inflammation leading to organ injuries. Thus, Brennan *et al.* reported that the removal of excess TNF from the sites of inflammation can treat the immune disorders caused by inflammatory responses (Brennan et al., 1989, Inhibitory effect of TNF-αantibodies on synovial cell interleukin-1 production in rheumatoid arthritis. Lancet 2, 244-247).

Feldmann *et al.* reported that TNF plays a particularly important role in the regulation of a cascade of pathogenic events in RA, Crohn's disease, psoriasis and other diseases, exemplified by the rapid induction of cytokines, such as IL-1β and IL-6 (Feldmann et al., 2002, Discovery of TNF-α as a therapeutic target in rheumatoid arthritis: preclinical and clinical studies. Joint Bone Spine 69, 12-18). TNF is over-expressed particularly in animal models of RA (Keffer et al., 1991, Transgenic mice expressing human tumor necrosis factor: a predictive genetic model of arthritis. EMBO J 10, 4025-4031). It has been suggested that a dramatic reduction in serum IL-6 concentrations occurred within 1 day of infliximab therapy was a direct effect of TNF neutralization (Charles et al., 1999, Regulation of cytokines, cytokine inhibitors, and acute-phase proteins following anti-TNF- α therapy in rheumatoid arthritis. J Immunol 163, 1521-1528).

Many biologics with inhibition of TNF-α have been produced, such as adalimumab, etanercept, infliximab, DMARDs (for example, methotrexate), or NSAIDs. Due to fast drug efficacy, dramatic effects and well immune toleration, these biologics have become main stream market products. However, biologics are expensive, demanding intravenous administration and have adverse effects, such as risks of inducing undesired immune responses or infection. Developing a drug which is safe for the immune system is challenging.

Bupleurum is a genus of plants used in Chinese medicine for reducing fever, relieving pain, eliminating toxins and reducing inflammation. In Chinese medicine, Bupleurum primarily treats fullness and discomfort in the chest, bitterness in the mouth, dryness in the throat, alternate spell of chill and fever, jaundice, hepatitis, enterogastritis and cholecystitis. The root of Bupleurum is usually used. It has been reported that saikosaponin, longispinogenin, sterols, lipid oil, isoflavones and sugars were isolated from the root of a plant of Bupleurum, and saikosaponin was determined as the chief component. Saikosaponin a and d have been proved to have medicinal effects (Liu S.U., et al., Development Diversity of Bupleurum, Journal of Agricultural Research of China, v.53, No. 2 (June, 2004)).

Bupleurum has been enthusiastically studied for hepatitis. However, there is no study or journal published implementing the application of Bupleurum to immunomodulation. The inventors have studied the extraction of Bupleurum, wherein the active ingredient of the extract was identified, and the relationship between the active ingredient and TNF-α or IL-6 *in vivo* was determined. Finally, a novel pharmaceutical composition with an immunomodulation has been developed.

### SUMMARY

A detailed description is given with reference to the accompanying drawings.

The present description provides a pharmaceutical composition with an immunomodulating function, comprising an extract of Bupleurum obtained by grinding the Bupleurum, adding the ground Bupleurum to a solvent and isolating the extract from the solvent, wherein the Bupleurum comprises *Bupleurum* krylovianum, *Bupleurum longiradiatum, Bupleurum smithii, Bupleurum pusillum, Bupleurum longicaule, Bupleurum salicifolium, Bupleurum scorzonerifolium* and Bupleurums with at least one of the nucleotide sequences selected from a group consisting of SEQ ID NOs. 1~6 and a nucleotide sequence with no more than 1% of the nucleotide sequence divergences of SEQ ID NOs. 1-6, or combinations thereof.

A pharmaceutical composition with an immunomodulating function, comprising kaerophyllin or cis-isomer as an active ingredient is also provided.

### BRIEF DESCRIPTION OF THE DRAWINGS

The present disclosure can be more fully understood by reading the subsequent detailed description and examples with references made to the accompanying drawings, wherein:

Fig. 1 is an HPLC chromatogram of the extracts of Bupleurum by a 95% ethanol aqueous solution.

Fig. 2 is an HPLC chromatogram of the extract of *Bupleurum* krylovianum by a 95% ethanol aqueous solution.

Fig. 3a is a ¹H NMR spectrum of the crystal (1) in Example 3, and Fig. 3b shows a ¹H NMR spectrum of the crystal (2) in Example 3.

Fig. 4a is an HPLC chromatogram of kaerophyllin, and Fig. 4b shows an HPLC chromatogram of isokaerophyllin.

Fig. 5 is the inhibition of TNF-α in an LPS-induced inflammatory animal model by different amounts of the ethanol extract of Bupleurum.

Fig. 6 is the inhibition of IL-6 in an LPS-induced inflammatory animal model by the amounts of the ethanol extract of Bupleurum.

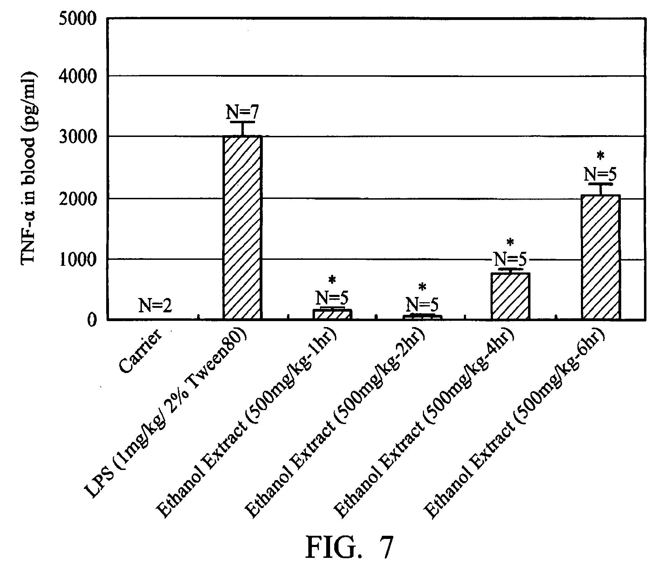

Fig. 7 is the inhibition of TNF-α in an LPS-induced inflammatory animal model by the ethanol extract of Bupleurum overtimes.

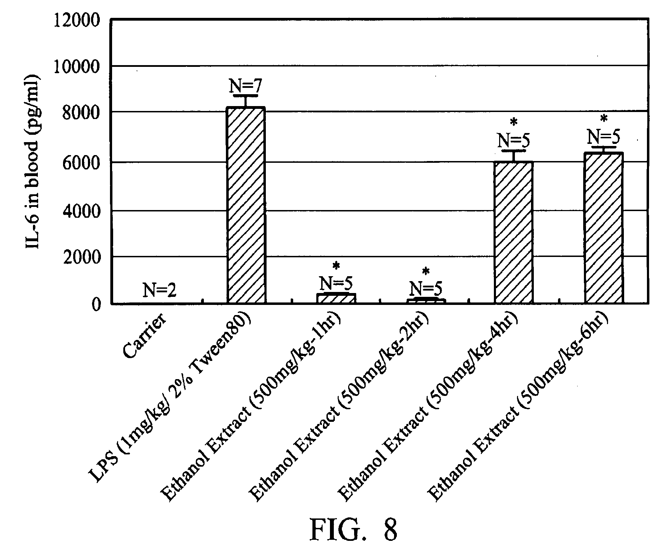

Fig. 8 is the inhibition of IL-6 in an LPS-induced inflammatory animal model by the ethanol extract of Bupleurum overtimes.

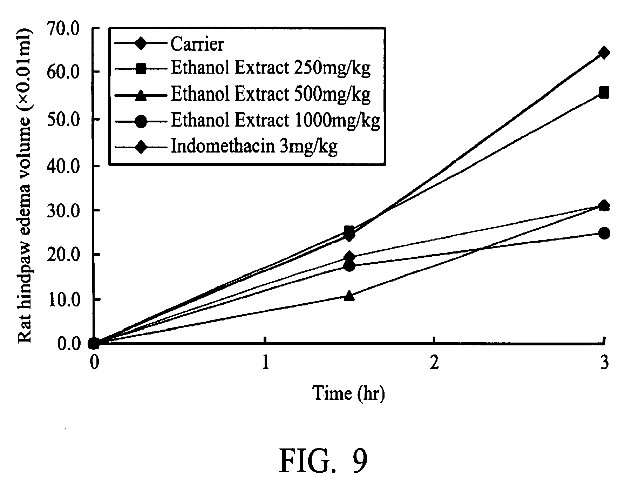

Fig. 9 is the inhibition of hindpaw edema in a carrageenan-induced hindpaw edema animal model by the ethanol extract of Bupleurum.

Fig. 10 is the inhibition of arthritis in an adjuvant-induced arthritis animal model by the ethanol extract of Bupleurum.

Fig. 11 is the inhibition of arthritis in an collagen-induced arthritis animal model by the ethanol extract of Bupleurum

Fig. 12 is the inhibition of TNF-α in a U937 cell line by kaerophyllin.

Fig. 13 is the inhibition of TNF-α in a U937 cell line by isokaerophyllin.

Fig. 14 is the inhibition of IL-6 in a U937 cell line by kaerophyllin.

Fig. 15 is the inhibition of IL-6 in a U937 cell line by isokaerophyllin.

### DETAILED DESCRIPTION

The invention provides use of an extract of *B. krylovianum* in the manufacture of a medicament for the treatment of arthritis or rheumatoid arthritis, wherein the extract of *B. krylovianum* is obtained by grinding *Bupleurum krylovianum,* adding the ground *Bupleurum krylovianum* to an ethanol aqueous solution and isolating the extract from the solvent..

Specifically, the Bupleurum comprises *Bupleurum krylovianum*.

While some medicinal plants are plentiful, some are not. Medicinal plants with similar morphology or names sometimes cause confusion, wherein they are interchanged. Traditional identification of medicinal plants require a skilled person, considering the plant morphology, and taste and performing microscopic examination. Once plants show similar morphology or lose their original taste, they are usually undistinguishable, which may lead to low medicinal efficacy or poison. Therefore, the Bupleurum is identified by chromosomal DNA polymorphism and nucleotide sequence divergence of internal transcribed spacer (ITS) of ribosomal RNA (rRNA). Thus, Bupleurum comprises the Bupleurum with a nucleotide sequence selected from a group consisting of: SEQ ID NOs. 1~6 and a nucleotide sequence with no more than 1% of the nucleotide sequence divergences of SEQ ID NOs. 1~6.

The Bupleurum can be extracted from the root of a plant or a whole plant. The "extraction" according to the invention can be solvent extraction. The "solvent extraction" is directed to a method comprising: adding a substrate of interest into a suitable solvent and extracting a target compound based on the solubility of components of the substrate in the solvent. In one example, the Bupleurum is ground and immerged in a solvent under room temperature for a period of time. The solvent is isolated and dried under room temperature to obtain an extract of the Bupleurum. In another example, the ground Bupleurum is added into a polar solvent and extracted after heating under reflux.

The solvent used in the invention can be C₁∼C₁₂ alcohols, C₂∼C₅ acetates, C₅∼C₆ alkanes, or combinations thereof, such as methanol, ethanol, propanol, isopropanol, butanol, 2-butyl alcohol, tert-butyl alcohol, 1,3-butandiol, 1,4butandiol, pentanol, isopentanol, 2,3-pentandiol, 2,4-pentandiol, cyclopentanol, hexanol, cyclohexanol, heptanol, octanol, nonanol, decanol, undecanol, dodecanol, ethyl acetate, propyl acetate, pentyl acetate, pentane, cyclopentane, hexane, cyclohexane, or combinations thereof, but are not limited thereto. In one example, ethanol, ethyl acetate and/or pentane is used as a solvent for extraction. In another example, an ethanol aqueous solution is used as a solvent for extraction. The concentration of ethanol can be 20%-95%, preferably 50%∼75% based on the ethanol aqueous solution.

The volume of the solvent can be more than 5 times that of the Bupleurum, or preferably 5~10 times.

The extraction is usually more than 2 hours, preferably 2~24 hours, more preferably 4~5 hours.

The extraction is usually under room temperature, preferably under room temperature to the boiling temperature of the ethanol aqueous solution, more preferably under the boiling temperature of the ethanol aqueous solution.

The extraction can further comprise a concentration process to concentrate and dry the extract after heating under reflux, wherein a solid or crystal is obtained. The extraction can be repeated several times to obtain a pure extract.

The extract of Bupleurum shows activities for inhibiting the expression of TNF-α or IL-6 *in vivo*, as shown in following examples, exhibiting immunomodulating function.

The extract of Bupleurum can be further isolated, such as by recrystallization. Kaerophyllin and cis-isomer have been isolated from Bupleurum. Kaerophyllin is known as one of the lignins, which is trans-(3,4-dimethoxybenzylidene)- β-(3,4-methylenedioxylbenzyl)-γ-butyrolactone, C₂₂H₂₂O₇, as presented in the formula (1). Kaerophiyllin usually forms transparent needle-like crystals at room temperature and has a melting point of 131°C∼132°C.

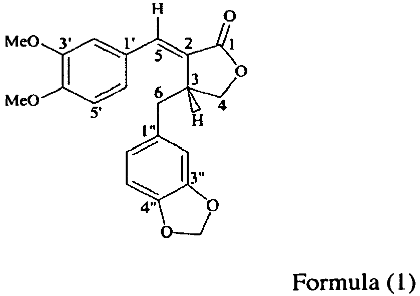

The extraction and identification of kaerophyllin from Bupleurum has been described in publications, such as Dou HS, et al (2000) Studies on method for the determination of kaerophyllin in Bupleurum smithii wolff Wolff var. parvifolium, China Journal of Chinese Materia Medica. 2000 Aug;25(8):488-9 ; Shang-Ming Yuan, et al (2000) Analysis of kaerophyllin in 4 kinds of Saiko by HPLC, Northwest Pharmaceutical Journal. 2000 15(04); Estevez-Braun A. et al., 1994, antibiotic activity and absolute configuration of 8S-Heptadeca- 2(Z),9(Z)-diene- 4,6-diyne-1,8-diol from Bupleurum salicifolium, J. Natural Products, 57, 1178-1182 ; and Estevez-Braun A. et al., 1995, Busaliol and Busalicifol, two new tetrahydrofuran lignans from Bupleurum salicifolium, J. Natural Products, 58, 887-892.

It has been discovered that kaerophyllin often exists in a mixture with cis-isomer. The cis-isomer of kaerophyllin is a transparent square crystal with a melting point of 146.5°C -148°C, which is cis-(3,4-dimethoxybenzylidene)-β-(3,4-methylenedioxylbenzyl)-γ-butyrolactone, C₂₂H₂₂O₇, as presented in the formula (2).

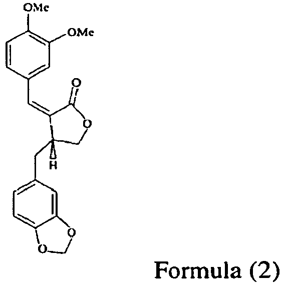

Currently, there is no references to prove that kaerophyllin and cis-isomer have biological functions. However, in the following examples, kaerophyllin and the cis-isomer show an effect on the expression of TNF-α or IL-6 *in vivo*. Therefore, kaerophyllin and the cis-isomer have an immunomodulating function *in vivo*.

Therefore, the pharmaceutical composition with immunomodulation can be applied for treating, alleviating or preventing disorders or symptoms relating to the function of TNF-α or IL-6, such as septic shock, sepsis, ischemic reperfusion, mycobacterial infection, meningitis, psoriasis, congestive heart failure, cachexia, transplant rejection, cutaneous T-cell lymphoma, angiogenesis, autoimmune disorders, dermatitis, Crohn's disease, colitis, osteoarthritis and rheumatoid arthritis, ankylosing spondylitis, psoriatic arthritis, adult Still's disease, uveitis, Wegener's granulomatosis, Behcehe disease, Sjogren's syndrome, sarcoidosis, polymyositis, dermatomyositis, multiple sclerosis, sciatica, periodontal disease, adult immunodeficiency syndrome, non-insulin-dependent diabetes mellitus (NIDDM), systematic lupus erythematosus (SLE), glaucoma, idiopathic pulmonary fibrosis (IPF), bronchopulmotary dysplasia, retinopathy, systematic sclerosis, osteoporosis, renal ischemia, myocardial infarction, stroke, ischemic stroke, nephritis, hepatitis, glomerulonephritis, atopic dermatitis, vasculitis, allergy, seasonal allergic rhinitis, reversible airway obstruction, adult respiratory distress syndrome, asthma, chronic obstructive pulmonary disease (COPD), bronchitis, or combinations thereof, but are not limited thereto.

The pharmaceutical composition may further comprise pharmaceutical acceptable carriers and/or additives in an appropriate percentage, which is well known in the art.

The pharmaceutical composition can be administrated intravenously, intramuscularly, orally or subcutaneously, and preferably orally. The administration can be performed in multiple dosages over a period of time. The regime can be designed according to pharmaceutical tests.

[Example 1] HPLC analysis of Bupleurum

The roots of several Bupleurum species were ground. 0.5g portions of the ground Bupleurum were individually added to 25 mL of a 95% ethanol aqueous solution and vibrated overnight. After concentrated and dried, 95% of ethanol solution was added to make a final volume of 2 mL. The solution was analyzed under high pressure liquid chromatography (HPLC) analysis.

### HPLC analysis:

Column: SymmetryShield^{™}(Waters), ^{RP}18, 5 µm, 250x4.6mmID
Mobile phase: A: H₂O, B: acetonitrile, C: methanol.
Detection: UV/ λ=237nm.

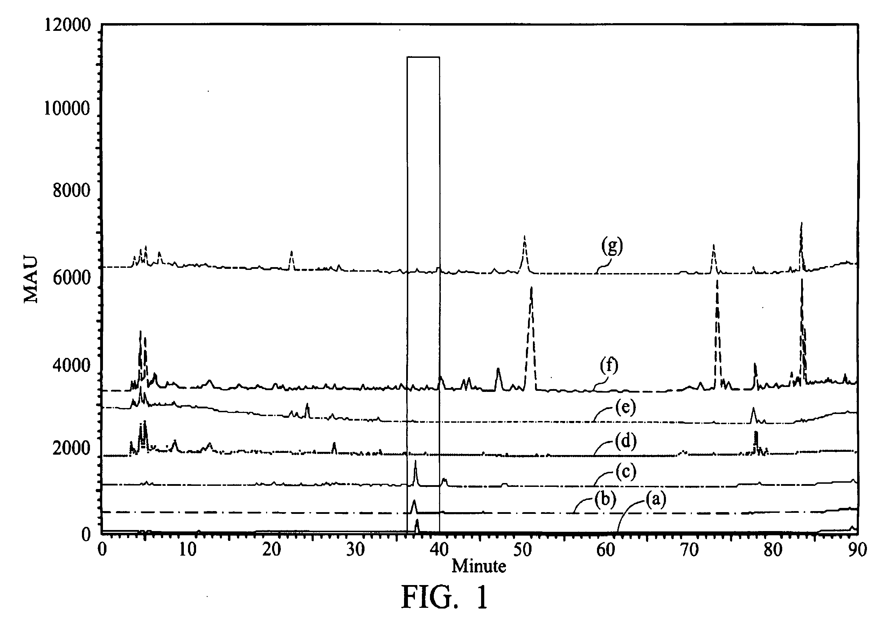

The HPLC chromatogram with the peak of kaerophyllin (standard, Line (a) in Fig. 1) was selected, wherein Line (b) is from a Bupleurum with a nucleotide sequence of SEQ ID NO. 1 (the sequencing is described in Example 18), Line (c) is from *Bupleurum* krylovianum with a nucleotide sequence of SEQ ID NO. 2, Line (d) is from *Bupleurum longiradiatum* with a nucleotide sequence of SEQ ID NO. 3, Line (e) is from Bupleurum with a nucleotide sequence of SEQ ID NO. 4, Line (f) is from Bupleurum with a nucleotide sequence of SEQ ID NO. 5, and Line (g) is from Bupleurum with a nucleotide sequence of SEQ ID NO. 6.

[Example 2] Extraction of *Bupleurum* krylovianum

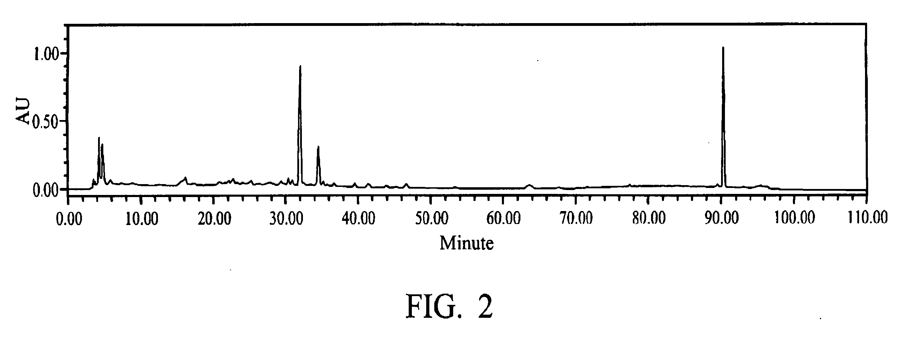

2kg of the ground *Bupleurum* krylovianum selected in Example 1 was added into 16L of a 95% ethanol aqueous solution. After 5 hours at room temperature, the solution was concentrated to obtain an extract with a 6.7±0.1% extraction yield. The extract was then analyzed under HPLC analysis. The resulting chromatogram shows peaks between 30-40 minutes (Fig. 2).

### HPLC analysis:

Column: SymmetryShield^{™}(Waters), ^{RP}18, 5 µm, 250x4.6mmID
Mobile phase: A: H₂O, B: acetonitrile, C: methanol.
Detection: UV/ λ=237nm.

[Example 3] Identification of active ingredients

18.5kg of the ground *Bupleurum krylovianum* selected in Example 1 was added into 25L of methanol. After 4 hours under room temperature, the solution was dried and concentrated to obtain 18.5kg of residues. 500mg of the residues was purified by using column chromatography (stationary phase: SiO₂, mobile phase: hexane, ethyl acetate and methanol with ratio of 6/4/1, 3/2/1 and 0/0/1) and elutes were collected separately. Each elutes was fractionated by thin layer chromatography (TLC) and 12 fractions were collected, respectively. Each of the 12 fractions was added to methanol to make a final concentration of 1mg/ml and analyzed by the HPLC analysis of Example 1. A chromatogram with the peak of kaerophyllin (standard) was selected. The selected fraction was further purified by the column chromatography and an extract with higher purity was isolated. The extract was further crystallized to obtain white transparent crystals (1).

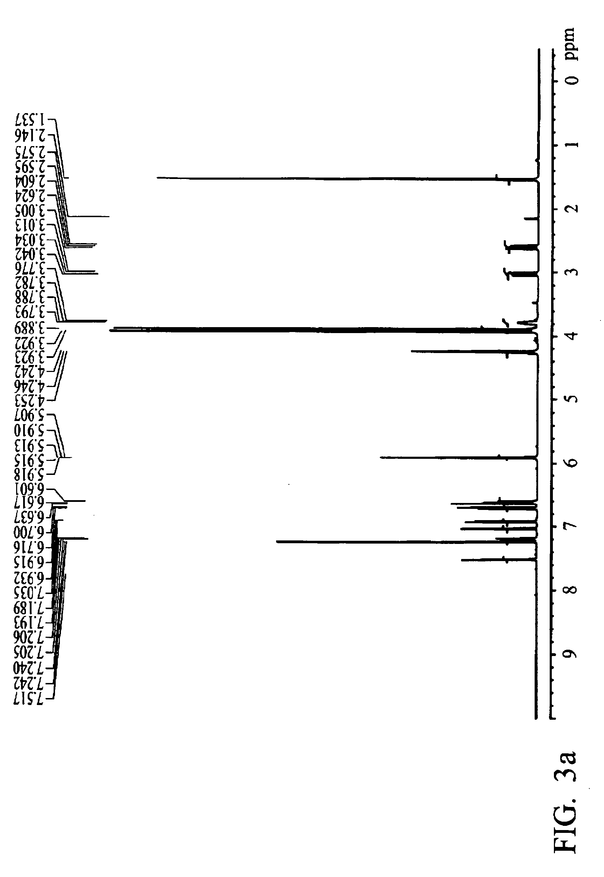

The crystal (1) was studied by ¹H-NMR, ¹³C-NMR and MS spectroscopy. As the result in Table 1, Table 2 and Fig. 3a shows, there were chemical shifts at 6-8 ppm and 2.5-4 ppm, suggesting that there were C₃ and C₆ lignins. Comparing the information of the kaerophyllin described in Wen-Liang C. et al., 2003, Immunosuppressive flavones and lignans from Bupleurum scorzonerifolium, Phytochemistry 64, 1375-1379, the crystal (1) were identified as kaerophyllin.

**[Table 1] ¹H-NMR data of kaerophyllin in Wen-Liang C. et al. and the crystal (1) of Example 3**

| Location | *δ* H mult of kaerophyllin in Wen-Liang C. *et al.* | *δ* H mult of the crystal (1) of Example 3 |
|---|---|---|
| 3 | 3.78m | 3.78 |
| 4α | 4.24m | 4.24 |
| 4*β* | 4.24m | 4.24 |
| 5 | 7.50s | 7.51 |
| 6*α* | 2.58dd(14.4, 10.3) | 2.59 |
| 6*β* | 3.01dd(14.4,4.1) | 3.01 |
| 2' | 7.02d(1.5) | 7.03 |
| 5' | 6.91d(8.4) | 6.91 |
| 6' | 7.19dd(8.4, 1.5) | 7.19 |
| 2" | 6.62d(1.5) | 6.61 |
| 5" | 6.69d(7.8) | 6.67 |
| 6" | 6.59dd(7.8, 1.5) | 6.60 |
| 3'-OMe | 3.88s | 3.88 |
| 4'-OMe | 3.91s | 3.92 |
| OCH₂O | 5.90d(1.5) | 5.90 |

**[Table 2] ¹³C-NMR (CDCl₃) data of kaerophyllin in Wen-Liang C. et al. and the crystal (1) of Example 3**

| Location | *δ* C of kaerophyllin in Wen-Liang C. *et al.* | δC of the crystal (1) of Example 3 |
|---|---|---|
| 1 | 172.56 | 172.57 |
| 2 | 125.58 | 125.73 |
| 3 | 39.60 | 39.72 |
| 4 | 69.46 | 69.53 |
| 5 | 137.39 | 137.43 |
| 6 | 37.53 | 37.50 |
| 1' | 126.82 | 126.97 |
| 2' | 112.87 | 113.06 |
| 3' | 150.65 | 150.80 |
| 4' | 149.05 | 149.21 |
| 5' | 111.25 | 111.40 |
| 6' | 123.54 | 123.54 |
| 1" | 131.43 | 131.54 |
| 2" | 108.91 | 109.06 |
| 3" | 147.91 | 148.03 |
| 4" | 146.49 | 146.61 |
| 5" | 108.41 | 108.50 |
| 6" | 121.89 | 121.97 |
| 3'-OMe | 55.93 | 56.05 |
| 4'-OMe | 55.96 | 56.05 |
| OCH₂O | 101.02 | 101.10 |

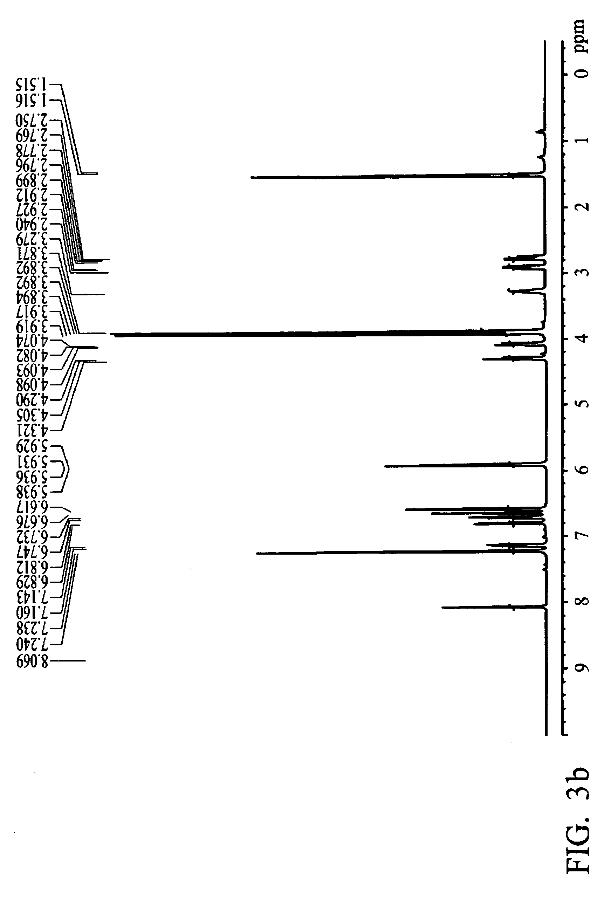

The selected fraction in Example 3 was recrystallized in hexane to obtain transparent square crystals (2). The crystal was studied by ¹H-NMR, ¹³C-NMR and MS spectroscopy and the results are shown in Table 3, Table 4 and Fig. 3b. Comparing the information of the isokaerophyllin described in Wen-Liang C. et al., 2003, Immunosuppressive flavones and lignans from Bupleurum scorzonerifolium, Phytochemistry 64, 1375-1379, it was identified that the crystal (2) was isokaerophyllin.

**[Table 3] ¹H-NMR data of isokaerophyllin in Wen-Liang C. et al. and the crystal (2) of Example 3**

| Location | *δ* H mult of isokaerophyllin in Wen-Liang C. *et al.* | δH mult of the crystal (2) of Example 3 |
|---|---|---|
| 3 | 3.28m | 3.28 |
| 4*α* | 4.09 | 4.09 |
| 4*β* | 4.30m | 4.3 |
| 5 | 6.61s | 6.61 |
| 6*α* | 2.77dd | 2.76 |
| 6*β* | 2.92dd | 2.92 |
| 2' | 8.07d | 8.07 |
| 5' | 6.82d | 6.81 |
| 6' | 7.15dd | 7.14 |
| 2" | 6.68d | 6.67 |
| 5" | 6.74d | 6.74 |
| 6" | 6.62dd | 6.61 |
| 3'-OMe | 3.89s | 3.89 |
| 4'-OMe | 3.92s | 3.91 |
| OCH₂O | 5.93d | 5.93 |

**[Table 4] ¹³C-NMR (CDCl₃) data of isokaerophyllin and the crystal (2) of Example 3**

| Location | *δ* C of isokaerophyllin in Wen-Liang C. *et al.* | δC of the crystal (2) of Example 3 |
|---|---|---|
| 1 | 172.56 | 172.57 |
| 2 | 125.58 | 125.73 |
| 3 | 39.6 | 39.72 |
| 4 | 69.46 | 69.53 |
| 5 | 137.39 | 137.43 |
| 6 | 37.53 | 37.6 |
| 1' | 126.82 | 126.97 |
| 2' | 112.87 | 113.06 |
| 3' | 150.65 | 150.8 |
| 4' | 149.05 | 149.21 |
| 5' | 111.25 | 111.4 |
| 6' | 123.54 | 123.54 |
| 1" | 131.43 | 131.54 |
| 2" | 108.91 | 109.06 |
| 3" | 147.91 | 148.03 |
| 4" | 146.49 | 146.61 |
| 5" | 108.41 | 108.5 |
| 6" | 121.89 | 121.97 |
| 3'-OMe | 55.93 | 56.05 |
| 4'-OMe | 55.96 | 56.05 |
| OCH₂O | 101.02 | 101.1 |

The white crystal (1) and transparent square crystal (2) of Example 3 were studied under HPLC analysis, which comprised:
Column: ODS 3V,
Mobile phase: CH₃OH/H₂O (0.1 %H3PO₄), gradient elution,
Flow rate: 1.0ml/min, and
Detection: UV/ λ =310nm.

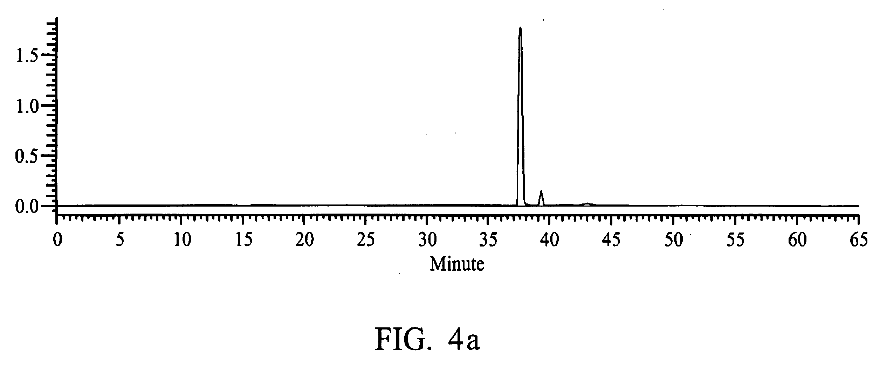

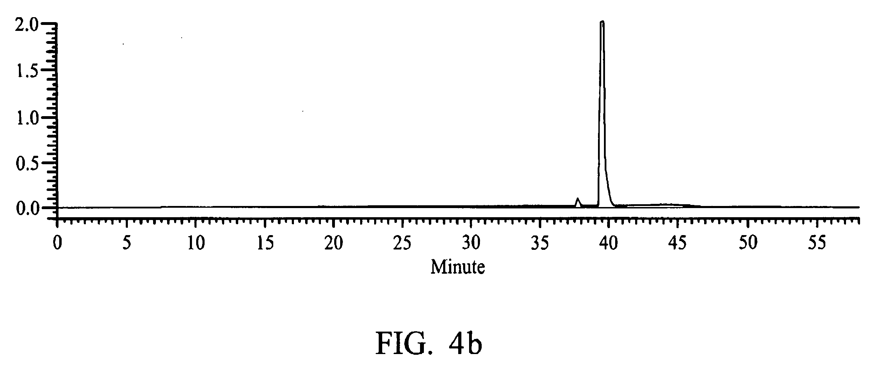

The resulting chromatograms showed that the white crystal (1) (kaerophyllin) had a peak at about 37 minutes (Fig. 4a) and the transparent square crystal (2) (isokaerophyllin) had a peak at about 40 minutes (Fig. 4b).

[Example 4] Inhibition of the extracts by different concentrations of ethanol extracts

200g of the ground *Bupleurum* krylovianum selected in Example 1 was equally divided and added to a four 1L round bottom flask. Each flask was added ethanol aqueous solutions with 20%, 50%, 75% and 95% concentrations, individually. After heating under reflux for 2 hours, each of the solutions was dried and concentrated to obtain extracts.

BALB/c mice were fed with the extracts in an amount of 500mg/kg, respectively. After 2 hours, the mice were intraperitoneally injected with 1mg/kg of lipopolysaccharide (LPS) in phosphate buffered saline (PBS). Blood was collected after 1.5 hours. The concentration of TNF-α and IL-6 in the mice serum was quantified by ELISA (R&D System®). The inhibition rates (%) of TNF-α and IL-6 in the serum were calculated based on the concentration of TNF-α and IL-6 in the serum before the mice were fed with the extracts. The results are shown in Table 5.

**[Table 5]**

| Ethanol concentration for extraction | Inhibition of TNF-*α* (%) | Inhibition of IL-6 (%) |
|---|---|---|
| 25% | 57.7 | 26.2 |
| 50% | 92.8 | 81.7 |
| 75% | 92.4 | 74.0 |
| 95% | 71.4 | 40.3 |

[Example 5] Inhibition of the extracts by different solvent extractions

(i) Extraction by 50% ethanol: 50g of the ground *Bupleurum* krylovianum selected in Example 1 and 500ml of 50% ethanol aqueous solution were added to a 1L round bottom flask. After heating under reflux for 2 hours, the solution was dried and concentrated to obtain an extract (a). The extract (a) was used in the following *in vivo* experiments.

(ii) Extraction by hexane: 50g of the ground *Bupleurum* krylovianum selected in Example 1 and 500ml of 50% ethanol aqueous solution were added to a 1L round bottom flask. The flask was heated under reflux for 2 hours and added 100% hexane in an equal amount of the solution in the flask. The step was repeated three times. The solution in the flask was dried and concentrated to obtain an extract (b). The extract (b) was used in the following *in vivo* experiments.

(iii) Extraction by ethyl acetate: 50g of the ground *Bupleurum* krylovianum selected in Example 1 and 500ml of 50% ethanol aqueous solution were added to a 1L round bottom flask The flask was heated under reflux for 2 hours and added ethyl acetate in an equal amount of the solution in the flask. The step was repeated three times. The solution in the flask was dried and concentrated to obtain an extract (c). The extract (c) was used in the following *in vivo* experiments.

(iv) *In vivo* experiments: BALB/c mice were fed with the extracts (a), (b) and (c) in an amount of 500mg/kg, respectively. After 2 hours, the mice were intraperitoneally injected with 1mg/kg of LPS in PBS. Blood was collected after 1.5 hours. The concentration of TNF-α and IL-6 in the mice serum was quantified by ELISA (R&D System®). The inhibition rates (%) of TNF-α and IL-6 in the serum were calculated based on the concentration of TNF-α and IL-6 in the serum before the mice were fed with the extracts. The results are shown in Table 6.

**[Table 6]**

| Solvent extraction | Extracts | Inhibition of TNF-*α* (%) | Inhibition of IL-6(%) |
|---|---|---|---|
| 50% ethanol | (a) | 92.8 | 81.7 |
| 50% ethanol + hexane | (b) | 99.3 | 78.4 |
| 50% ethanol + ethyl acetate | (c) | 98.6 | 83.0 |

[Example 6] The isolation of kaerophyllin by different solvent extractions

5g of the ground *Bupleurum krylovianum* selected in Example 1 was equally divided and added to five 1L round bottom flasks. Each flask was added 10 ml of methanol, ethanol, isopropanol, butanol and decanol, respectively. Each flask was ultrasound vibrated for 10 minutes and the extracts were collected individually. The volume for each extract was adjusted to 10 ml. Meanwhile, standards were established as follows: 0.3 mg/ml, 0.27 mg/ml, 0.21 mg/ml, 0.15, 0.09 mg/ml, 0.06 mg/ml and 0.03 mg/ml of kaerophyllin. The amounts of kaerophyllin in the extracts were determined as shown in Table 7.

**[Table 7]**

| Solvent for extraction | The amount of kaerophyllin in the extracts (%) |
|---|---|
| Methanol | 0.32 |
| Ethanol | 0.30 |
| Isopropanol | 0.21 |
| Butanol | 0.08 |
| Decanol | 0.10 |

[Example 7] Inhibition effects by different extraction times

2.0 kg of the ground *Bupleurum* krylovianum selected in Example 1 and 16.3L of 50% ethanol solution were added to a 20L round bottom flask under heating with reflux. The solutions were collected separately after 2, 3, 4 and 5 hours and then dried and concentrated to obtain extracts. Meanwhile, standards were established as follows: 0.3 mg/ml, 0.27 mg/ml, 0.21 mg/ml, 0.15, 0.09 mg/ml, 0.06 mg/ml and 0.03 mg/ml of kaerophyllin The amounts of kaerophyllin in the extracts were determined as shown in Table 8.

In addition, BALB/c mice were fed with the extracts in an amount of 500mg/kg. respectively. After 2 hours, the mice were intraperitoneally injected with 1mg/kg of LPS in PBS. Blood was collected after 1.5 hours. The concentration of TNF-α and IL-6 in the mice serum was quantified by ELISA (R&D System®). The inhibition rates (%) of TNF- α and IL-6 in the serum were calculated based on the concentration of TNF-α and IL-6 in the serum before the mice were fed with the extracts. The results are shown in Table 8.

**[Table 8]**

| Extraction time | The amount of kaerophyllin in the extract | Inhibition of TNF-*α* (%) | Inhibition of IL-6 (%) |
|---|---|---|---|
| 2hr | 1.10 | 84.4 | -34.7 |
| 3hr | 1.08 | 70.5 | -6.8 |
| 4hr | 1.06 | 100.0 | 46.0 |
| 5hr | 1.06 | 99.7 | -0.9 |

[Example 8] Effect of the ethanol extract on LPS-induced inflammatory response in a mouse model

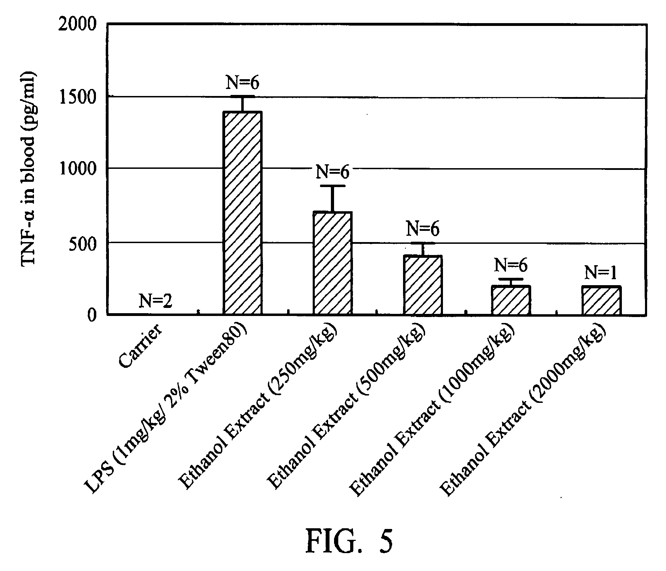

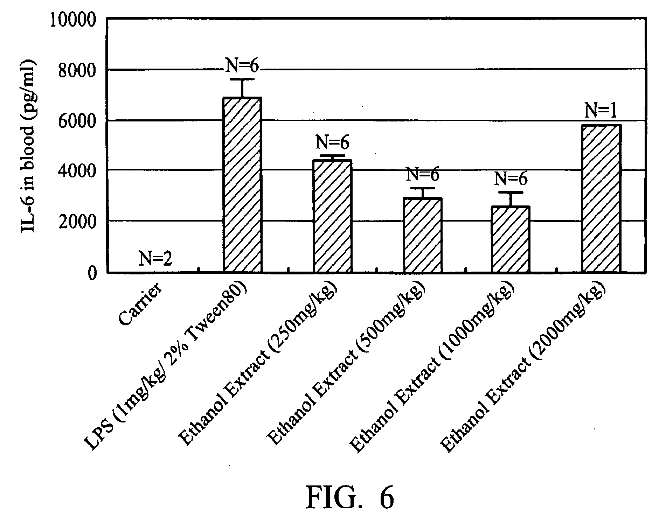

BALB/c mice were fed with the extract (a) in Example 5 (i) in an amount of 250mg/kg, 500 mg/kg, 1000mg/kg and 2000 mg/kg, respectively. After 2 hours, the mice were intraperitoneally injected with 1mg/kg of LPS in PBS. Blood was collected after 1.5 hours. The concentration of TNF- α and IL-6 in the mice serum was quantified by ELISA (R&D System®). The inhibition rates (%) of TNF-α and IL-6 in the serum were calculated based on the concentration of TNF-α and IL-6 in the serum before the mice were fed with the extracts. The results are shown in Fig. 5 and 6. The control was fed with 10ml/kg of 2% Tween 80 as a carrier.

[Example 9] Effect of the ethanol extract on LPS-induced inflammatory response in a mouse model at different administration times

BALB/c mice were fed with the extract (a) in Example 5 (i) in an amount of 500 mg/kg. The mice were intraperitoneally injected with 1mg/kg of LPS in PBS respectively at 1, 2, 4 and 6 hours after being fed with the extract. Blood was collected after 1.5 hours. The concentration of TNF- α and IL-6 in the mice serum was quantified by ELISA (R&D System^{®} ). The inhibition rates (%) of TNF-α and IL-6 in the serum were calculated based on the concentration of TNF-α and IL-6 in the serum before the mice were fed with the extract. The results are shown in Fig. 5 and 6. The control was fed with 10ml/kg of 2% Tween 80 as a carrier.

[Example 10] Effect of the ethanol extract on carrageenan induced hindpaw edema in a rat model

Long-Evan rats were fed with the extract (a) in Example 5 (i) in an amount of 250mg/kg, 500 mg/kg, 1000mg/kg, respectively. The positive control was fed with 3mg/kg of indomethacin (NSAID). The negative control was fed with 10ml/kg of 2% Tween 80 as a carrier After 1 hour, the rats were injected 0.1ml of 1% carrageenan on the left hindpaw. The volume of the rat's left hindpaw was measured by plethysmometer (Stoelting) at the 0, 1.5th, and 3rd hour after injection. The inhibition was calculated by the formula :

Inhibition (%)=(Nt-Nv)/Nv × 100

In the formula, Nt represents the left hindpaw volume of the rat fed with the ethanol extract; Nv represents the left hindpaw of the rat fed with the carrier.

The inhibition was negative when the ethanol extract showed anti-inflammatory effects.

**[Table 9]**

| Administration | Inhibition at 0 hour (%) | Inhibition at the 1.5th hour (%) | Inhibition at the 3rd hour (%) |
|---|---|---|---|
| Carrier | 0 | 0 | 0 |
| 250mg/kg of ethanol extract | 0 | 3 | 14 |
| 500 mg/kg of ethanol extract | 0 | 55 | 52 |
| 1000 mg/kg of ethanol extract | 0 | 29 | 62 |
| 3 mg/kg of indomethacin | 0 | 21 | 52 |

[Example 11] Effect of the ethanol extract on adjuvant induced arthritis in a rat model

The rats were categorized into 5 groups. Each group had 5 rats. The rats were injected 50µl of *Mycobacterium butyricum* in squalene at 3 points on the root of the tail (total injection: 50µl/rat). The rats in 5 groups were immediately fed with 500mg/kg of the extract (a) in Example 5 (i), 1000mg/kg of the extract (a) in Example 5 (i), 3mg/kg of indomethacin, 0.1mg/kg of dexamethasone and 10ml/kg of 2% Tween 80 (carrier),, respectively. The symptoms of the arthritis in each group were estimated according to the scores:
0: The feet were not red and swelling;
1: The feet were slightly red and swelling or one toe joint was red and swelling;
2: The feet were apparently red and swelling or more than two toe joints were red and swelling;
3: The hindpaw was incapable of function for walking;
4: The ankle joint was incapable of moving.

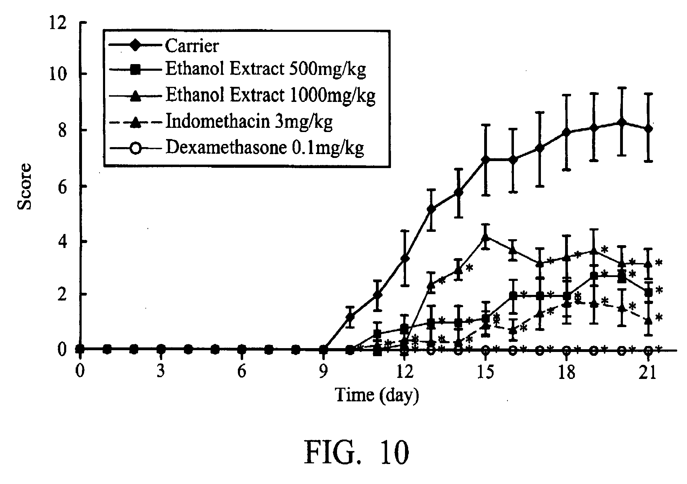

The result is shown in Fig. 10. The ethanol extract showed inhibition of arthritis, compared to the carrier group (p<0.05). The administration of indomethacin and doxamethasone was the positive control.

[Example 12] Effect of the ethanol extract on collagen induced arthritis in a rat model

2mg/ml of a fetus bovine type II collagen was emulsified with an equal amount of a complete Freund's adjuvant (CFA) or incomplete Freund's adjuvant (IFA) by homogenization (IKA, RW20 DZM.n.). The rats were categorized into 4 groups. Each group had 8 rats. Each rat was intracutaneously injected 50 µg of the collagen and CFA emulsion. At the eighth day, each rat was injected 100 µg of the collagen and IFA emulsion to induce arthritis. The rats in 4 groups were immediately fed with 50mg/kg of the extract (a) in Example 5 (i), 30mg/kg of celebrex (NSNID), 10ml/kg of 1% carboxymethyl cellulose (CMC) (carrier) and nothing, respectively. The symptoms of the arthritis in each group were estimated according to the scores:
0: The foot was not red and swelling;
1: The foot was slightly red and swelling or one toe joint was red and swelling;
2: The foot was apparently red and swelling or more than two toe joints were red and swelling;
3: The hindpaw was incapable of function for walking;
4: The ankle joint was incapable of moving.

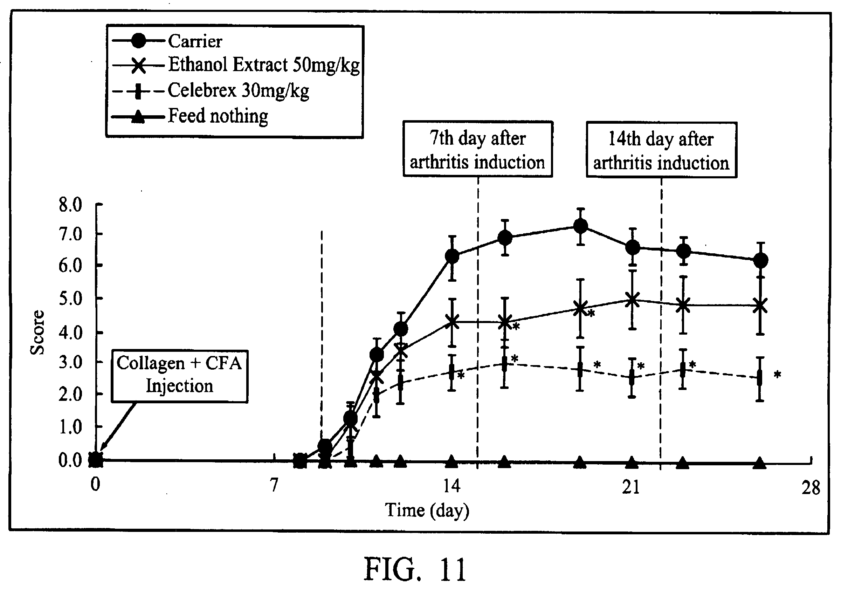

The result showed that 50mg/kg of the ethanol extract can cause apparent inhibition of arthritis (Fig. 11). The administration of celebrex was the positive control, and CMC was the negative control. The group fed nothing was a blank test.

[Example 13] Effect of kaerophyllin and the cis-isomer on TNF- α (for reference)

The cell line, U937 (human monocyte cell line), was incubated in RPMI medium containing 50 ng/ml PMA (phorbol 12-myristate 13-acetate) (Sigma) and 10% fetal bovine serum (Moor et. al., Roswell Park Memorial Institute) for 24 hours. The cell line was then moved to the RPMI medium without PMA for further 48 hours. The activated U937 was seeded into a 96-well plate in a concentration of 1.6×10⁵ cell/well. The wells were added 12.5 µg/ml, 25 µg/ml, 50 µg/ml and 100 µg/ml of kaerophyllin and 1 µg/ml, 3 µg/ml, 10 µg/ml and 30 µg/ml of isokaerphyllin and 10 µl of buffer, respectively. Each well was adjusted to a final volume of 190 µl. The plate was reacted under 37°C for 30 minutes. Each well was added 10 µl of 20 µg/ml LPS to stimulate cells. After 4 hours under 37°C, the plate was centrifuged to collect the supernatants.

The amount of TNF-α in the supernatant was measured by ELISA (R&D System®) based on the control (DMSO). Meanwhile, the cell viability was measured by MTT assay (Sigma).

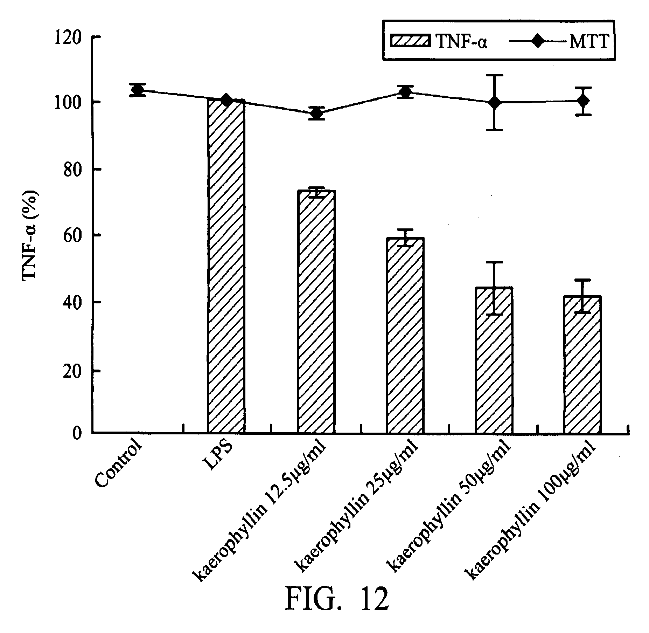

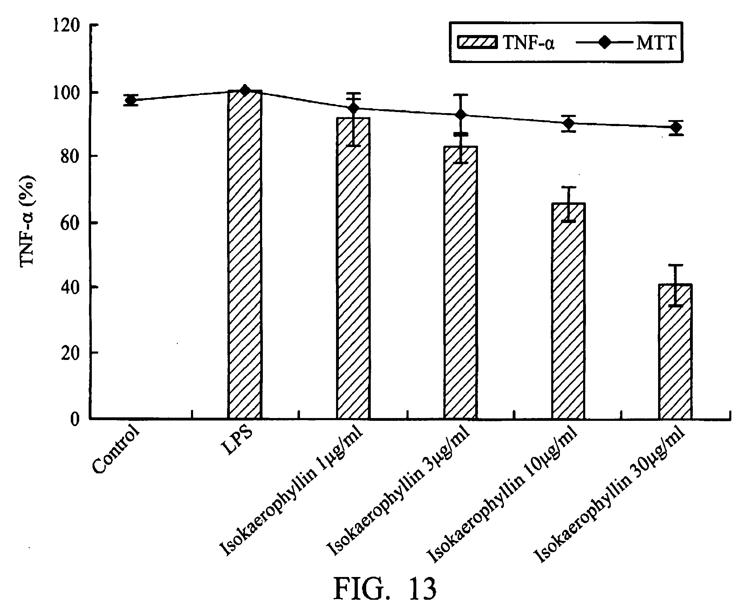

The results showed that kaerophyllin and the cis-isomer had effects to inhibit the excretion of TNF-α. The IC₅₀ value of kaerophyllin was 44±5 µg/ml (Fig. 12). The IC₅₀ value of isokaerophyllin was 18±4 µg/ml (Fig. 13).

[Example 14] Effect of kaerophyllin and the cis-isomer on IL-6 (for reference)

The cell line, U937, was cultured like Example 13. The activated U937 was /s then seeded in a 96-well plate in a concentration of 1.6×10⁵ cell/well. The wells were added 6.3 µg/ml, 12.5 g g/ml, 25 µg/ml and 50 µg/ml of kaerophyllin and 3.8 µg/ml, 7.5 µg/ml, 15 µg/ml and 30 µg/ml of isokaerphyllin and 10 µl of buffer, respectively. Each well was adjusted to a final volume of 190 µl. The plate was put under 37°C for 30 minutes. Each well was added 10µl of 20 µg/ml LPS to stimulate cells. After 16-18 hours under 37°C, the plate was centrifuged to collect the supernatants.

The amount of IL-6 in the supernatant was measured by ELISA (R&D System®) based on the control (DMSO). Meanwhile, the cell viability was measured by MTT assay (Sigma).

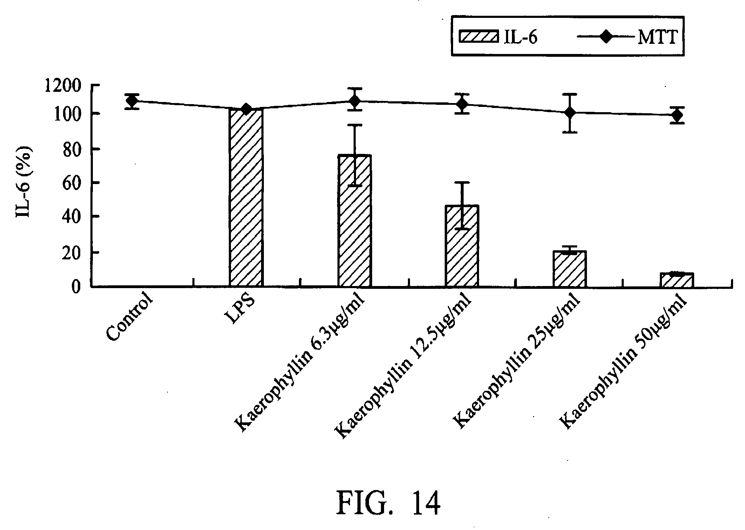

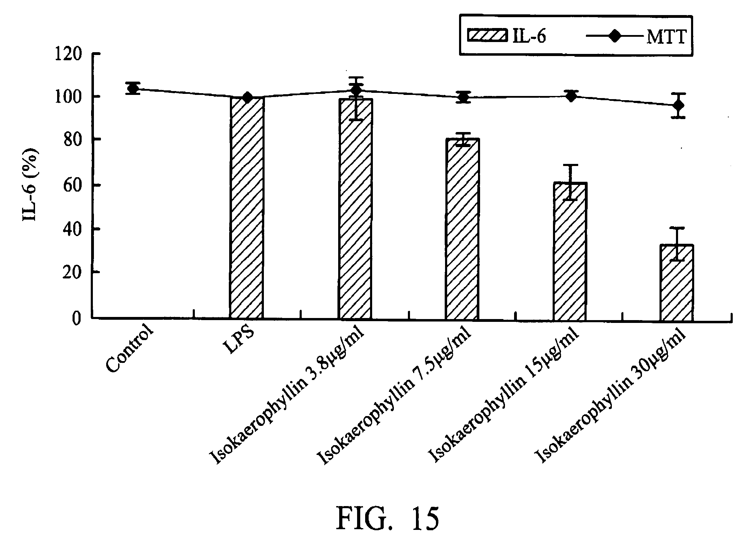

The results showed that kaerophyllin and the isomer had effects to inhibit the excretion of IL-6. The IC₅₀ value of kaerophyllin was 13±2 µg/ml (Fig. 14). The IC₅₀ value of isokaerophyllin was 24 ±6 µg/ml (Fig. 15).

[Example 15] Effect of the ethanol extract in an enteritis mouse model

The BABL/c mice were categorized into 4 groups. Each group had 6 rats. One group was injected 50% ethanol and others were injected 1.75 mg of trinitrobenzene sulfonate (TNBS) (Sigma) into the colon (4 cm up from the anus). The TNBS-treated groups were immediately fed with 100mg/kg of the extract (a) in Example 5 (i), 0.6mg/kg of dexamethasone (DEX) and nothing, respectively. After 48 hours, the colon (6 cm up from the anus) of each mice was excised and the colon was immerged in PBS for 2 hours. The colon infusion was measured for the amounts of TNF-α, IL-6 and G-CSF. The inhibition of TNF-α, IL-6 and G-CSF was calculated based on the TNBS-treated group without being fed anything. The results are shown in Table 10, in which a negative value represents enhanced excretion of TNF-α, IL-6 and G-CSF.

**[Table 10]**

| Group | Inhibition of TNF-*α*(%) | Inhibition of IL-6 (%) | Inhibition of G-CSF (%) |
|---|---|---|---|
| 50% ethanol | 74 | 96 | 93 |
| 1.75mg of TNBS | 0 | 0 | 0 |
| 100mg/kg of the ethanol extract + 1.75mg of TNBS | 50 | 43 | 60 |
| 0.6mg/kg of DEX + 1.75mg of TNBS | 9 | -55 | -38 |

[Example 16] Effect of Bupleurum species on LPS induced inflammatory response in a mouse model

Six species of Bupleurum selected from Example 1 were individually pulverized and screened by a sieve with 5 meshes.

50g of each ground Bupleurum and 400mL of 50% ethanol aqueous solution were added into six 2L round bottom flasks. The flasks were heated under reflux for 2 hours. The solution was collected. After dried and concentrated, the extracts (1)-(6) were obtained, which correspond to the Lines (b)-(g) of the Bupleurum species in Example 1.

BALB/c mice were fed with 1000mg/kg of the extract (1) and 500mg/kg of the extracts (2)-(6), respectively. After 2 hours, the mice were intraperitoneally injected with 1mg/kg of LPS in PBS. Blood was collected after 1.5 hours. The concentration of TNF-α and IL-6 in the mice serum was quantified by ELISA (R&D System®). The inhibition rates (%) of TNF-α and IL-6 in the serum were calculated based on the concentration of TNF-α and IL-6 in the serum of the mice without being fed the extracts. The results are shown in Table 11.

**[Table 11]**

| Extracts | Inhibition of TNF-*α* (%) | Inhibition of IL-6 (%) |
|---|---|---|
| Extracts (1) | 44 | -6 |
| Extracts (2) | 99 | 50 |
| Extracts (3) | 75 | 42 |
| Extracts (4) | 25 | 29 |
| Extracts (5) | 27 | 51 |
| Extracts (6) | 34 | 11 |

[Example 17] Identification of the Bupleurum by ITS sequencing

0.5∼1g of the six Bupleurum (b)~(g) in Example 1 were individually pulverized with liquid nitrogen. The powder was poured into centrifugal tubes containing 7~10 ml of a CTAB buffer and well mixed. The tube was vibrated 30~60 minutes under 70°C. The tubes were added 5ml of chloroform and well mixed. The tubes were centrifuged at 8,000 rpm for 5 minutes at 4°C. The supernatants were removed. The precipitates were centrifuged at 12,000 rpm for 20 minutes at 4°C. The supernatants were removed again. Each tubes was added 3ml of 1.2M NaCl containing RNase. The tubes were vibrated for 30~60 min. at 37 °C, added 3ml of chloroform and well mixed. The tubes were centrifuged at 8,000 rpm for 5 min. at 4°C. The supernatants were moved to new 1.5ml microcentrifuge tubes and added isopropanol of 0.6 times that of the volume. The tubes were put in a -20°C refrigerator to precipitate overnight. After centrifuged, the precipitates were washed by 75% ethanol once and dried. The precipitates were resolved with 50 µl of TE. The solution was under PCR with primers to amplify the DNA sequences of the Bupleurum species. The DNA sequences of the Bupleurum (b)~(g) are shown in the sequence list in accordance with SEQ ID NOs. 1~6.
## Claims

1. Use of an extract of *B. krylovianum* in the manufacture of a medicament for the treatment of arthritis or rheumatoid arthritis, wherein the extract of *B. krylovianum* is obtained by grinding *Bupleurum krylovianum,* adding the ground *Bupleurum krylovianum* to an ethanol aqueous solution and isolating the extract from the solvent.

2. The use as claimed in claim 1, wherein the volume of the solvent is 5∼10 times that of *B. krylovianum.*

3. The use as claimed in claim 1, wherein the time for extraction is 2 to 24 hours.

4. The use as claimed in claim 1, wherein the extract is further heated under reflux.

5. The use as claimed in claim 1, wherein the extraction is under room temperature to a boiling temperature of the ethanol aqueous solution.

6. The use as claimed in claim 1, wherein the extract comprises kaerophyllin or cis-isomer as an active ingredient.

## Patentansprüche

1. Verwendung eines Extrakts von *B. krylovianum* in der Herstellung eines Medikaments zur Behandlung von Arthritis oder rheumatoider Arthritis, wobei der Extrakt von *B. krylovianum* durch Mahlen von *Bupleurum krylovianum,* Geben des gemahlenen *Bupleurum krylovianum* zu einer wässrigen Ethanol-Lösung und Isolieren des Extrakts aus dem Lösungsmittel erhalten wird.

2. Verwendung wie in Anspruch 1 beansprucht, wobei das Volumen des Lösungsmittels das 5- bis 10-fache von *B. krylovianum* ist.

3. Verwendung wie in Anspruch 1 beansprucht, wobei die Extraktionszeit 2 bis 24 Stunden beträgt.

4. Verwendung wie in Anspruch 1 beansprucht, wobei der Extrakt ferner unter Rückfluss erwärmt wird.

5. Verwendung wie in Anspruch 1 beansprucht, wobei die Extraktion unter Raumtemperatur bis zu einer Siedetemperatur der wässrigen Ethanol-Lösung durchgeführt wird.

6. Verwendung wie in Anspruch 1 beansprucht, wobei der Extrakt Kaerophyllin oder cis-Isomer als einen Wirkstoff umfasst.

## Revendications

1. Utilisation d'un extrait de *B. krylovianum* dans la fabrication d'un médicament pour le traitement de l'arthrite ou de la polyarthrite rhumatôide, où l'extrait de *B. krylovianum* est obtenu par broyage de *Bupleurum krylovianum,* addition du *Bupleurum krylovianum* broyé à une solution aqueuse d'éthanol et isolement de l'extrait à partir du solvant.

2. Utilisation selon la revendication 1, où le volume du solvant est 5-10 fois celui de *B. krylovianum.*

3. Utilisation selon la revendication 1, où la durée pour l'extraction est 2 à 24 h.

4. Utilisation selon la revendication 1, où l'extrait est en outre chauffé à reflux.

5. Utilisation selon la revendication 1, où l'extraction est à la température ambiante à une température d'ébullition de la solution aqueuse d'éthanol.

6. Utilisation selon la revendication 1, où l'extrait comprend de la kaerophylline ou un isomère cis comme ingrédient actif.
